(19) Europäisches Patentamt / European Patent Office / Office européen des brevets

(11) **EP 3 066 436 B1**

(12) **EUROPEAN PATENT SPECIFICATION**

(45) Date of publication and mention
of the grant of the patent:
**22.01.2025 Bulletin 2025/04**

(21) Application number: **14806133.6**

(22) Date of filing: **10.11.2014**

(51) International Patent Classification (IPC):
*G01J 3/50* $^{(2006.01)}$      *G01J 3/46* $^{(2006.01)}$

(52) Cooperative Patent Classification (CPC):
**G01J 3/504; G01J 3/462; G01J 3/463**

(86) International application number:
**PCT/US2014/064786**

(87) International publication number:
**WO 2015/070134 (14.05.2015 Gazette 2015/19)**

(54) **TEXTURE ANALYSIS OF A COATED SURFACE USING ELECTROSTATICS CALCULATIONS**

TEXTURANALYSE EINER BESCHICHTETEN OBERFLÄCHE MITTELS ELEKTROSTATIKBERECHNUNGEN

ANALYSE DE TEXTURE D'UNE SURFACE REVÊTUE UTILISANT DES CALCULS D'ÉLECTROSTATIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.11.2013 US 201361901493 P**

(43) Date of publication of application:
**14.09.2016 Bulletin 2016/37**

(73) Proprietor: **PPG Industries Ohio, Inc.**
**Cleveland, OH 44111 (US)**

(72) Inventor: **NORRIS, Alison M.**
**Avon, Ohio 44011 (US)**

(74) Representative: **f & e patent**
**Braunsberger Feld 29**
**51429 Bergisch Gladbach (DE)**

(56) References cited:
**US-A1- 2008 235 224      US-B1- 6 539 325**

**Description**

CROSS REFERENCE TO RELATED APPLICATION

The present application claims priority to U.S. Provisional Application No. 61/901,493, filed on November 8, 2013.

FIELD OF THE INVENTION

The present invention generally relates to the use of electrostatics methods to relate spectral reflectances or colorimetric information from spectrophotometric angles and/or incident light sources to identify the proper pigment(s) to match both the texture and/or gonioapparent effect(s) occurring within an unknown target coating.

BACKGROUND OF THE INVENTION

In order to provide a proper color match via formulation or search engines (or a visual process) it is ideal to determine the correct pigmentation of the sample. It is clearly sensible that utilizing the same pigments or appropriate offsets of those pigments to an original sample will allow a formulation or search process to arrive at an apparent optimum solution. It is equally clear that excluding those pigments, either deliberately or inadvertently, from availability will result in a less than optimal color match.

Several formulation engines and methodologies attempt to encompass pigment selection and formulation via various algorithms simultaneously. Historically, pigment identification packages and formulation engines largely have taken a "brute" force, guess and check type of method to provide formulations and pigment information to their users. The combinatorial approach, or brute force method, is a frequently used method in which nearly all available pigments are combined in all the various combinations available given an end number of pigments desired in the final match. The combinatorial approach may utilize the Kubelka-Munk equation or a derivative thereof to generate the various formulations. Although there have been some methods which restrict the usage of some pigments given certain conditions to optimize the engine's speed, the end result is these formula combinations are then validated against the sample and a selection (or a single) of formulas most nearly matching the sample are provided to the user. There are various forms of Delta E's or other colorimetric assessment algorithms used to determine the accuracy of the match compared to the sample.

The more elegant solutions require the user to submit a sample set of toners to a formulation engine, while the less elegant methods often select a predefined subset of toners to use. Neither of the approaches utilize a stepwise method and thus often result in non-optimal solutions. These methods have been typically burdensome for the users and lacked proper "intuition" to provide a streamlined method to a good solution for the user. Additionally, by the nature of this methodology, appropriate pigments necessary to match the sample can be excluded.

In a standard portable spectrophotometer, the incident light is generally set at an angle of forty-five (45) degrees from normal. The resulting spectral reflectances that can be gathered are generally in the same plane as the incident light and are on either side of the specular angle (equal and opposite angle to the incident light) as well as nearer to the incident light source itself. New portable spectrophotometric devices offer a vast multitude of angular color response (spectral reflectance) data. Besides the addition of several new angles, including azimuthal, or out-of-plane, angles, many instruments also offer additional light sources with different geometries from standard. By way of example, the incident light source of a second illuminator may be located at fifteen (15) degrees from normal. The plurality of combinations of incident light and angular response can be both too little and too much information to be handled at one time, generating an enormous amount of spectrophotometric data. There is however a lack of methods to efficiently handle and analyze all of these data in a purposeful way. On the other hand also the new spectrophotometric devices capture only a part of the spectral response of the analyzed sample for selected illumination conditions. Strategies have been recently developed using painted or virtual samples representing various textures and comparing those to unknown samples. These techniques require substantial user intervention and are significantly subjective which produces inconsistent results dependent upon the skill of the individual. Examples of the prior art can be found in documents US6539325 B1 and US20080235224.

A simplified approach using limited multiangle, multiplaner when available, spectral data with or without a camera, color or otherwise, that can produce improved and simplified results for pigment characterization and sample properties is preferable for speed and ease of use. Providing a simplified system which can accurately determine the pigmentation of a sample to be fed to a formulation engine or visual color matching process greatly improves speed and accuracy. Including the formulation engine in that same flexible system further improves performance, accuracy, and simplicity.

Thus, there is a need for systems and methods that may be used to efficiently evaluate all of the data and specific combinations of data from a spectrophotometer allowing in particular meaningful inferences regarding the texture and/or gonioapparent effect(s) of an analyzed unknown target coating. Therefore the present invention aims to provide means which overcome or at least amend all or at least some of the afore-mentioned deficiencies of the prior art and enable in particular an efficient use and evaluation of the data obtained from state of the art spectrophotometers, for a reliable, more precise color matching of target coatings having texture and/or gonioapparent effects. These objectives are attained by the computer-implemented method, the system, the apparatus and the non-transitory computer readable medium including software as described in the following.

SUMMARY OF THE INVENTION

In a first aspect, the present invention provides a computer implemented method as defined in claim 1.
In another aspect, the present invention is directed to a system as defined in claim 3.
In a further aspect, the present invention provides a non-transitory computer readable medium as defined in claim 5.

BRIEF DESCRIPTION OF THE DRAWINGS

FIG. 1 illustrates a process that calculates a pigmentation for a target complex coating making use of the present invention.
FIGS. 2 and 3 illustrate a pseudo-electric field.
FIG. 4 illustrates an example of the use of an electrostatic field using a specific combination of angles to predict whether or not a target coating will contain a gonioapparent effect.
FIG. 5 illustrates an example of an empirical correlation of two different angle electrostatics data compared in a pairwise manner versus whether the target coating contains a specific gonioapparent pigment.
FIG. 6 illustrates a different type of example of another form of an empirical correlation.
FIG. 7 illustrates a system which may be used to identify physical property attributes such as, reflective characteristics under different or identical lighting conditions which are visually and/or spectrophotometrically unique or distinguishable from one pigment to another, of a coating mixture of a target sample according to the present invention.

DETAILED DESCRIPTION OF THE INVENTION

While the description herein generally refers to paint or coating, it should be understood that the devices, systems and methods apply to other types of coatings, including stain and industrial coatings. The described embodiments of the invention should not be considered as limiting. A method consistent with the present invention may be practiced in a variety of fields such as the matching and/or coordination of apparel and fashion products.
The present invention may be used with or incorporated in a computer system that may be a standalone unit or include one or more remote terminals or devices in communication with a central computer via a network such as, for example, the Internet or an intranet. As such, the computer or "processor" and related components described herein may be a portion of a local computer system or a remote computer or an on-line system or combinations thereof. The database and software described herein may be stored in computer internal memory or in a non-transitory computer readable medium.
The present invention is directed generally to spectral analysis of coatings, and more particularly, but not by way of limitation, to devices, methods and systems for predicting and formulating a complex coating mixture containing metallic, pearlescent, and/or special effect pigments.
The present invention generally relates to a method and apparatus for identifying physical property attributes of cured complex coating (e.g., paint) mixtures using electrostatics data that are calculated as explained herein, using a processor, based on the spectral reflectance and colorimetric response from a spectrophotometer. As will be explained in more detail below, according to the present invention the individual spectral reflectances of the reflectance data being obtained at selected points in space (corresponding to individual reflection angles), which depend on the configuration of the device used to obtain the reflectance data, are treated as point charges. Accordingly, electrostatics data means within the context of the present invention any kind of data calculated from the obtained reflectance data applying said point charge analogy in combination with the established laws and concepts of electrostatics. Examples of such calculated electrostatic data include e.g. electrostatic force data, electric field data, electrical potentials etc.
The purpose of using an electrostatics methodology is multi-fold. First, in order to use all available angles within a given system, electrostatics may be used to create an alternate bi-directional reflectance distribution function ("BRDF")-type analysis. This type of analysis does not exclude any angles, but uses all angles to create a hemispherical "map" or "fingerprint" of a particular coating, texture or pigment type, whether gonioapparent or not. Second, electrostatics may be used to evaluate only specific combinations of angles in order to achieve purposeful manipulations. Similarly, this includes the specific exclusion of specific singular angles or combinations of angles when a particular texture or effect is being sought after as included or not included in a target coating. Third, electrostatics may be used to accommodate for and correct the potential assumption that the received spectral reflectance values are incorrect in some way. Some potential reasons for irregularity or abnormality of the spectral reflectance data, even if minor, may include incident light angle location, incident light fluctuation, aperture size, target coating surface non-uniformity, etc.

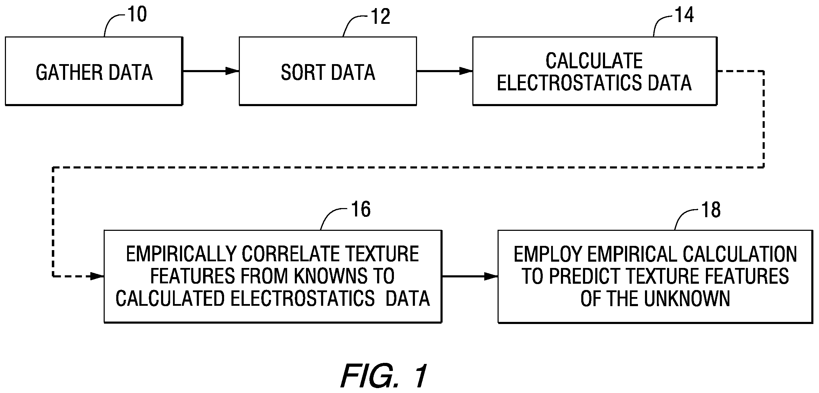

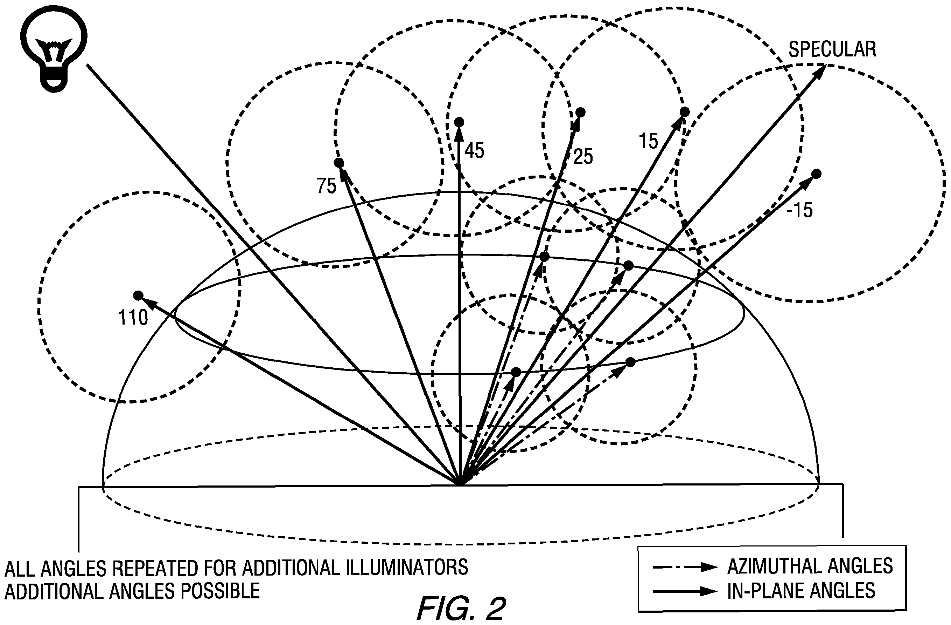

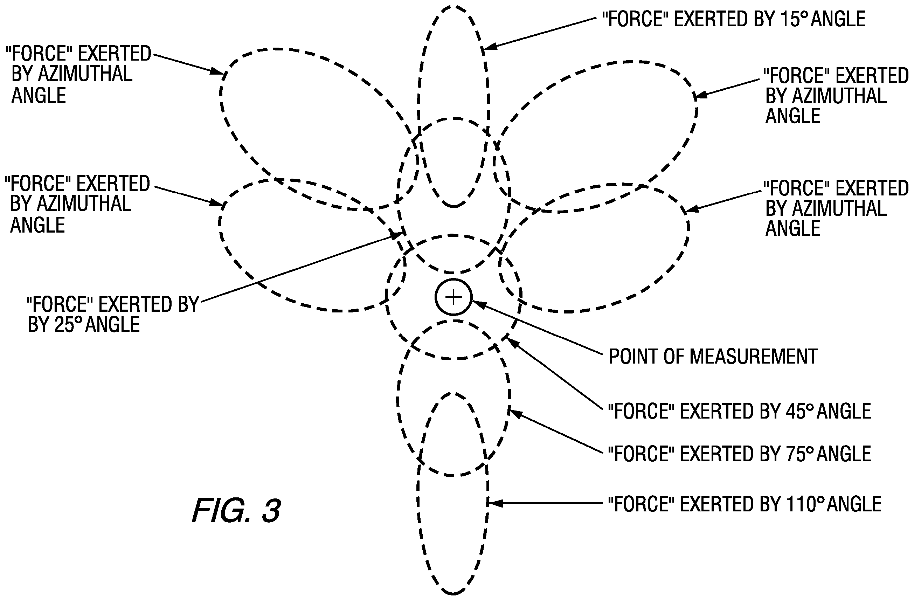

FIG. 1 illustrates a process that calculates a pigment for a target complex coating making use of the present invention. At step 10, reflectance data is gathered from a spectrophotometer and, at step 12, the data are sorted based on a secondary array of associated information. The primary array comes from the spectrophotometer. Secondary array is the sorted information. At step 14, electrostatics data is calculated. Herein, individual reflectances colorimetric information at specific physical angular ports, or angles in conjunction with the incident light angle will act as point charges. There can be one or multiple lights of incidence with which it is used in conjunction with the incident angle to enable a user to check and validate that the generated data is all in alignment and from the correct source (thus the multiple arrays). Each of such conceptualized point charges exerts an effect of influencing electrostatic "forces" on other reflectances across the physical layout of the respective measurement arrangement, for example, a portable spectrophotometer as shown in FIG. 2 and 3 schematically. A pseudo- electric field may be created as illustrated in FIGS. 2 ("standard" view) and 3 ("top down" view). Based on the number of point charges (reflectances and/or colorimetric information) that are desired for comparison at a single time, Coulomb's Law is used to define the electric field or force between point charges created by a single point charge or between multiple point charges. In a simplistic form, Coulomb's Law is as follows for two point charges:

$$F = \frac{Q_1 Q_2}{4\pi r^2 \varepsilon_0} \qquad \text{Equation (1)}$$

where $Q_1$ and $Q_2$ are the point electric charges, or in the case of texture analysis, the reflectance at a particular angle and wavelength and/or colorimetric, r is the distance between the two point charges on the surface of the sphere, and $\varepsilon_0 = 8.854187817 \times 10^{-12}$. If reflectance values are used as the point charge values, they are wavelength dependent. Therefore, the calculation occurs individually for each wavelength and is integrated at the end-usage point of the data.

In order to calculate the distance, r, between the two point charges, one possible solution is to use a portion of a great circle distance calculation by employing a special form of the Vincenty formula to calculate the spherical angle between the two point charges:

$$\Delta\hat{\sigma} = \arctan\left( \frac{\sqrt{\left(\cos\phi_f \sin\Delta\lambda\right)^2 + \left(\cos\phi_s \sin\phi_f - \sin\phi_s \cos\phi_f \cos\Delta\lambda\right)^2}}{\sin\phi_s \sin\phi_f + \cos\phi_s \cos\phi_f \cos\Delta\lambda} \right) \qquad \text{Equation (2)}$$

where $\phi_f$, $\lambda_f$, and $\phi_s$, $\lambda_s$ are coordinate locations, in radians, of the point charges on the surface of a sphere. The distance, r, then follows as:

$$r = R\,\Delta\sigma \qquad \text{Equation (3)}$$

where R is the radius of the sphere. In the case of a portable spectrophotometer, this may be assumed to equal 1 or may be accurately measured. The value for R does not change because it is likely the same or a similar spectrophotometer model will be used, and thus R remains constant. If it is expected that R may, in fact change then R may be appropriately measured and accounted for in Equation 3.

In various embodiments, in order to use the special form of the Vincenty formula, spatial coordinates, or latitude and longitude, for the point charges (reflectances and/or colorimetric data) may be assigned. The spatial coordinate assignments may be created using the physical angular layout of the spectrophotometer. By way of example, the spatial coordinates of the point charges may become coordinates that are functions of at least one of: (1) a version of the angle reflected light, which may be dependent upon the incident light angle; (2) an indication of in-plane or out-of-plane as well as location within that plane; (3) spectral reflectance value and/or colorimetric data at a particular wavelength. While a two point charge example is shown for the particular form of Coulomb's Law, any single or multiple combinations of angular point charges may be used with various forms of Coulomb's Law. Additionally, in examples not part of the invention, other electrostatics calculations, such as electric field, electric potential, etc. may be used in place of Coulomb's Law force equation. Further, these other electrostatics calculations may be used in conjunction with Coulomb's law force equation. The potential for singular, paired, triplicate, etc. comparisons may provide a sizeable amount of information, which may be used to identify texture. The incident light angle, either from normal or from parallel, may be used within the coordinate definitions of other angular data. This may be useful when working with data from multiple incident light angles or when including a comparison of the data received from the same physical receptor on the instrument, however the incident light came from multiple angles.

When using spectral reflectance data, the calculation occurs individually for each wavelength. Statistics, such as for example mean, median, and sum may be used to create a singular value out of multiple wavelength calculated electrostatics values. An individual specific wavelength or wavelengths may be compared between electrostatics analysis. The value of such a situation is to focus on the wavelength or wavelengths of maximum reflectance, and potentially secondary maximum reflectance, where a majority of color and/or texture information is visibly perceived within the visible spectrum. An analysis of shifting maximum reflectances by wavelength may also be completed using electrostatics analysis.

At step 16 of FIG. 1, the calculated electrostatics values from the colorimetric data and/or spectral reflectance data is further empirically correlated to known characteristics in order to identify textures, primary flake types, or other appearance information in complex coating mixtures. To employ an empirical method, the electrostatics data points (force, field, potential, etc.) are calculated for an empirical dataset and all desired combinations of angles, representative of the expected mixtures and colors that will need to be handled in everyday situations. The empirical data set is used to create a predictive correlation: $y = f(x)$, where $y$ represents the desired characteristic for identification or a qualitative question regarding the target coating, and $f(x)$ is some function of $x$'s, where $x$ is one or multiple variables using the electrostatics calculated values from a specific set or multiple sets of angular considerations. It may be desirable to limit the angular comparison sets to the specific sets that are most feature-defining for the particular characteristic that is being identified. The resulting function may be linear or non-linear as defined by the empirical data set.

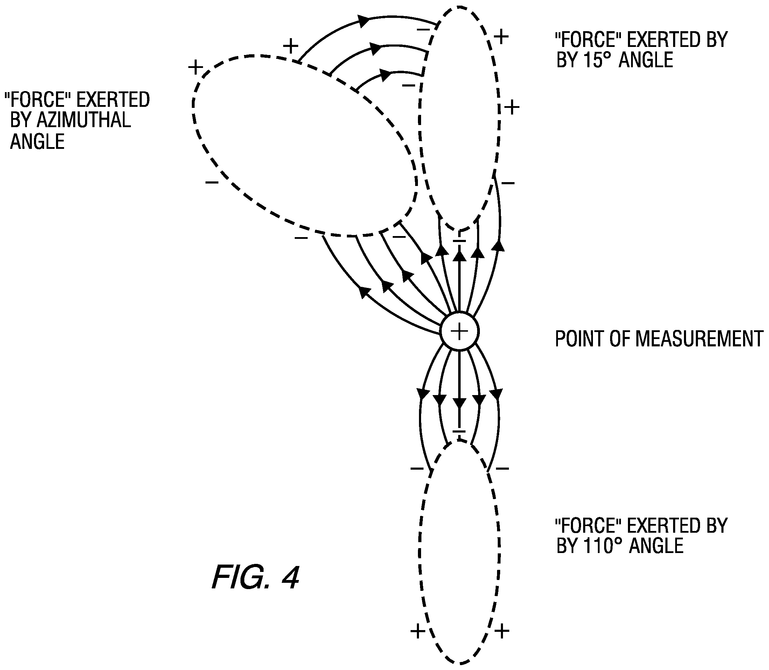

FIG. 4 illustrates an example of the use of an electrostatic field using a specific combination of angles to predict whether or not a target coating will contain a gonioapparent effect.

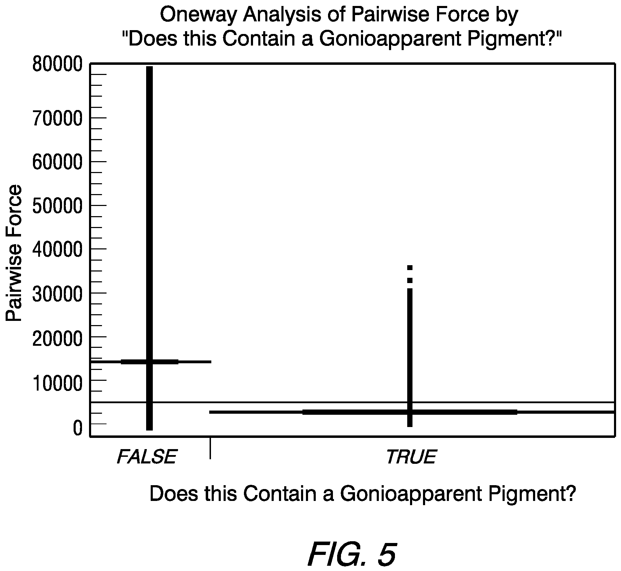

FIG. 5 illustrates an example of an empirical correlation of two different angle electrostatics data compared in a pairwise manner versus whether the target coating contains a specific gonioapparent pigment. In this example, a calculated electrostatics value of the same pairwise angular combination resulting in a value over roughly 5500 indicates a greater likelihood of containing gonioapparent versus not containing a gonioapparent pigment.

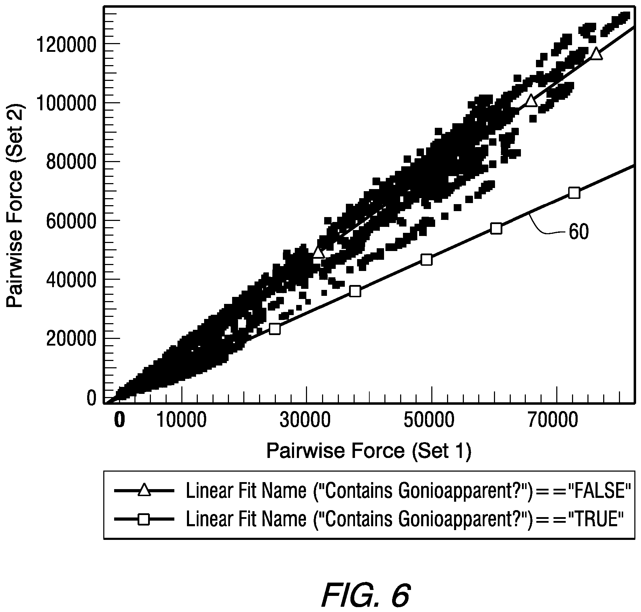

FIG. 6 illustrates a different type of example of another form of an empirical correlation. In this example scenario, two sets of pairwise angular comparisons using calculated electrostatics data are graphed versus one another. To use the correlation, the target coating's values may be input in the electrostatics calculations and whichever linear correlation's resultant graphical value it is more closely aligned to offers a higher likelihood of that effect being present. In many cases, the degree of likelihood may also be assessed based on a residual calculation from the correlations that were originally empirically determined. In such an example, a target coating calculated electrostatics value closer to correlation line 60 ("TRUE") indicates a higher likelihood of the target coating containing the specific gonioapparent effect versus not containing that effect.

Once an empirical correlation has been determined, at step 18 of FIG. 1 it is used to derive the predicted value for the target coating. This may be achieved by using the target coating's values for the $x$'s (electrostatic force, field, potential, etc.) and calculating the answer for $y$ (the texture effect). While examples have been given herein for the content of a gonioapparent pigment, the present invention may derive a result as specific as which gonioapparent pigment at which size flake of that pigment by iteratively choosing the most important single angles or combinations of angles for the electrostatics calculations and empirical correlations. The choice of angular comparisons and to what level they are combined may be used to create the best possible empirical correlation. Empirical correlations may also be improved by including other non-electrostatics information, for example singular angle colorimetric data.

The quality of the overall "map," or "fingerprint," approach and the quality of the empirical correlation approach may be dependent upon the quality of the input data. The quality of the input data may be dependent upon the quality of the instrumentation and the quality of the data set used to create a set of knowns for the overall map or the empirical correlation. While any quality of data from an instrument or an empirical data set will result in an answer, the answer may be improved with the use of a high quality instrument and a widely varied, high quality empirical data set.

The entire set of calculations described herein is used in conjunction with a processor in order to facilitate the choice of specific angle combination as well as accommodate the volume of calculations required in order to derive and then use an empirical correlation using electrostatics data.

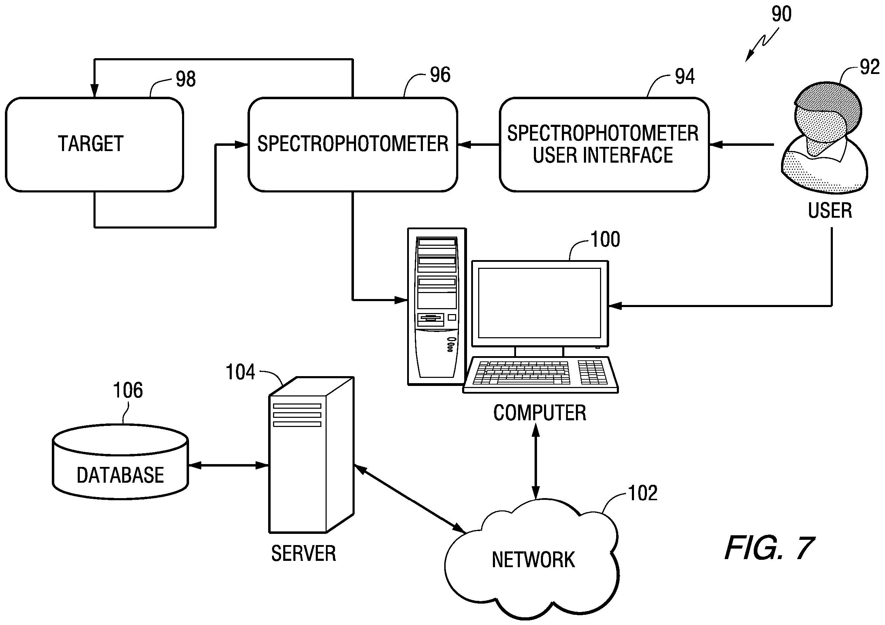

FIG. 7 illustrates a system 90 which may be used to identify physical property attributes such as, reflective characteristics under different or identical lighting conditions which are visually and/or spectrophotometrically unique or distinguishable from one pigment to another, of a coating mixture of a target sample according to the present invention. A user 92 may utilize a user interface 94, such as a graphical user interface, to operate a spectrophotometer 96 to measure the properties of a target sample 98. The data from the spectrophotometer 96 may be transferred to a computer 100, such as a personal computer, a mobile device, or any type of processor. The computer 100 may be in communication, via a network 102, with a server 104. The network 102 may be any type of network, such as the Internet, a local area network, an intranet, or a wireless network. The server 104 is in communication with a database 106 that may store the data and information that is used by the methods of the present invention for comparison purposes. The database 106 may be utilized in, for example, a client server environment or in, for example, a web based environment such as a cloud computing environment. Various steps of the methods of the present invention may be performed by the computer 100 and/or the server 104.

In another aspect, the invention is implemented as a non-transitory computer readable medium containing software for causing a computer or computer system to perform the method described above. The software can include various modules that are used to enable a processor and a user interface to perform the methods described herein.

It will be readily appreciated by those skilled in the art that modifications may be made to the invention without departing from the concepts disclosed in the forgoing description. Accordingly, the particular embodiments described in detail herein are illustrative only and are not limiting to the scope of the invention, which is defined by the appended claims.

## Claims

1. A computer implemented method, comprising:

   obtaining, using a processor, spectral reflectance data from a target coating at a plurality of individual reflection angles, wherein the spectral reflectance data is obtained using a spectrophotometer at selected points in space corresponding to the individual reflection angles;
   calculating, using the processor, pseudo-electrostatics data from the spectral reflectance data, wherein calculating the pseudo-electrostatics data comprises treating the spectral reflectance data obtained at the individual reflection angles as point charges and applying Coulomb's law, and wherein the calculation occurs individually for each wavelength; and generating based on the pseudo-electrostatics data, using the processor, a coating pigmentation that is the same or substantially similar in appearance to the target coating,

   wherein generating the coating pigmentation comprises empirically correlating the pseudo-electrostatics data calculated for the target coating to a plurality of known pseudo-electrostatics data calculated for an empirical dataset and predicting at least one texture feature of the target coating based on the correlating.

2. The computer implemented method of claim 1, wherein predicting at least one texture feature of the target coating based on the correlating comprises predicting, using at least one empirical calculation, at least one texture feature of the target coating based on the correlating.

3. A system, comprising:

   a database; and
   a processor in communication with the database and programmed to:

   obtain spectral reflectance data from a target coating at a plurality of individual reflection angles, wherein the spectral reflectance data is obtained using a spectrophotometer at selected points in space corresponding to the individual reflection angles;
   calculate pseudo-electrostatics data from the spectral reflectance data, wherein calculating the pseudo-electrostatics data comprises treating the spectral reflectance data obtained at the individual reflection angles as point charges and applying Coulomb's law, and wherein the calculation occurs individually for each wavelength; and
   generate based on the pseudo-electrostatics data a coating pigmentation that is the same or substantially similar in appearance to the target coating, wherein generating the coating pigmentation comprises empirically correlating the pseudo-electrostatics data calculated for the target coating to a plurality of known pseudo-electrostatics data calculated for an empirical dataset and predicting at least one texture feature of the target coating based on the correlating.

4. The system of claim 3, further comprising the spectrophotometer in communication with the processor.

**5.** A non-transitory computer readable medium including software for causing a processor to:

obtain spectral reflectance data from a target coating at a plurality of individual reflection angles, wherein the spectral reflectance data is obtained using a spectrophotometer at selected points in space corresponding to the individual reflection angles;
calculate pseudo-electrostatics data from the spectral reflectance data, wherein calculating the pseudo-electrostatics data comprises treating the spectral reflectance data obtained at the individual reflection angles as point charges and applying Coulomb's law, and wherein the calculation occurs individually for each wavelength; and
generate based on the pseudo-electrostatics data a coating pigmentation that is the same or substantially similar in appearance to the target coating, wherein generating the coating pigmentation comprises empirically correlating the pseudo-electrostatics data calculated for the target coating to a plurality of known pseudo-electrostatics data calculated for an empirical dataset and predicting at least one texture feature of the target coating based on the correlating.

**Patentansprüche**

**1.** Ein computerimplementiertes Verfahren, umfassend:

Erhalten, unter Verwendung eines Prozessors, spektraler Reflexionsdaten von einer Zielbeschichtung bei einer Vielzahl individueller Reflexionswinkel,
wobei die spektralen Reflexionsdaten unter Verwendung eines Spektrophotometers an ausgewählten Punkten im Raum entsprechend den individuellen Reflexionswinkeln erhalten werden;
Berechnen, unter Verwendung des Prozessors, pseudo-elektrostatischer Daten aus den spektralen Reflexionsdaten,
wobei das Berechnen der pseudo-elektrostatischen Daten ein Behandeln der spektralen Reflexionsdaten, die bei den individuellen Reflexionswinkeln erhalten wurden, als Punktladungen und ein Anwenden des Coulombschen Gesetzes umfasst, und wobei die Berechnung für jede Wellenlänge individuell erfolgt; und
Erzeugen, basierend auf den pseudo-elektrostatischen Daten, unter Verwendung des Prozessors, von einer Beschichtungspigmentierung die gleich oder im Wesentlichen ähnlich im Aussehen wie die Zielbeschichtung ist;
wobei das Erzeugen der Beschichtungspigmentierung ein empirisches Korrelieren der für die Zielbeschichtung berechneten pseudo-elektrostatischen Daten mit einer Vielzahl bekannter pseudo-elektrostatischer Daten, die für einen empirischen Datensatz berechnet wurden, und das Vorhersagen mindestens eines Texturmerkmals der Zielbeschichtung basierend auf der Korrelation umfasst.

**2.** Das computerimplementierte Verfahren nach Anspruch 1, wobei das Vorhersagen mindestens eines Texturmerkmals der Zielbeschichtung, basierend auf der Korrelation, ein Vorhersagen, unter Verwendung mindestens einer empirischen Berechnung, von mindestens einem Texturmerkmal der Zielbeschichtung, basierend auf der Korrelation, umfasst.

**3.** Ein System umfassend:

eine Datenbank; und
einen Prozessor, in Kommunikation mit der Datenbank und programmiert, um:

spektrale Reflexionsdaten von einer Zielbeschichtung bei einer Vielzahl von individuellen Reflexionswinkeln zu erhalten, wobei die spektralen Reflexionsdaten unter Verwendung eines Spektrophotometers an ausgewählten Punkten im Raum entsprechend den individuellen Reflexionswinkeln erhalten werden;
pseudo-elektrostatische Daten aus den spektralen Reflexionsdaten zu berechnen, wobei das Berechnen der pseudo-elektrostatischen Daten ein Behandeln der spektralen Reflexionsdaten, die bei den individuellen Reflexionswinkeln erhalten wurden, als Punktladungen und ein Anwenden des Coulombschen Gesetzes umfasst, und wobei die Berechnung individuell für jede Wellenlänge erfolgt; und
basierend auf den pseudo-elektrostatischen Daten eine Beschichtungspigmentierung zu erzeugen, die gleich oder im Wesentlichen ähnlich im Aussehen wie die Zielbeschichtung ist, wobei das Erzeugen der Beschichtungspigmentierung ein empirisches Korrelieren der für die Zielbeschichtung berechneten pseudo-elektrostatischen Daten mit einer Vielzahl bekannter pseudoelektrostatischer Daten, die für einen empirischen Datensatz berechnet wurden, und ein Vorhersagen mindestens eines Texturmerkmals der Zielbeschichtung basierend der Korrelation umfasst.

**4.** Das System nach Anspruch 3, ferner umfassend das Spektrophotometer, in Kommunikation mit dem Prozessor.

**5.** Ein nicht-flüchtiges computerlesbares Medium, enthaltend Software, um einen Prozessor zu veranlassen:

spektrale Reflexionsdaten von einer Zielbeschichtung bei einer Vielzahl individueller Reflexionswinkel zu erhalten, wobei die spektralen Reflexionsdaten unter Verwendung eines Spektrophotometers an ausgewählten Punkten im Raum entsprechend den individuellen Reflexionswinkeln erhalten werden; pseudo-elektrostatische Daten aus den spektralen Reflexionsdaten zu berechnen, wobei das Berechnen der pseudo-elektrostatischen Daten ein Behandeln der spektralen Reflexionsdaten, die bei den individuellen Reflexionswinkeln erhalten wurden, als Punktladungen und ein Anwenden des Coulombschen Gesetzes umfasst, und wobei die Berechnung individuell für jede Wellenlänge erfolgt; und basierend auf den pseudo-elektrostatischen Daten eine Beschichtungspigmentierung zu erzeugen, die gleich oder im Wesentlichen ähnlich im Aussehen wie die Zielbeschichtung ist, wobei das Erzeugen der Beschichtungspigmentierung ein empirisches Korrelieren der für die Zielbeschichtung berechneten pseudo-elektrostatischen Daten mit einer Vielzahl bekannter pseudo-elektrostatischer Daten, die für einen empirischen Datensatz berechnet wurden, und ein Vorhersagen mindestens eines Texturmerkmals der Zielbeschichtung basierend auf der Korrelation umfasst.

**Revendications**

**1.** Procédé informatique comprenant les étapes suivantes :

obtenir, en utilisant un processeur, des données de réflectance spectrale d'un revêtement cible selon une pluralité d'angles de réflexion individuels, dans lequel les données de réflectance spectrale sont obtenues en utilisant un spectrophotomètre en des points choisis de l'espace correspondant aux angles de réflexion individuels ; calculer, en utilisant le processeur, des données pseudo-électrostatiques à partir des données de réflectance spectrale, dans lequel le calcul des données pseudo-électrostatiques comprend le fait de traiter les données de réflectance spectrale obtenues aux angles de réflexion individuels comme des charges ponctuelles et d'appliquer la loi de Coulomb, et dans lequel le calcul se fait individuellement pour chaque longueur d'onde ; et générer d'après les données pseudo-électrostatiques, en utilisant le processeur, une pigmentation de revêtement qui est la même ou sensiblement similaire en apparence au revêtement cible, dans lequel la génération de la pigmentation de revêtement comprend le fait de corréler de manière empirique les données pseudo-électrostatiques calculées pour le revêtement cible à une pluralité de données pseudo-électrostatiques connues calculées pour un ensemble de données empirique et de prédire au moins une caractéristique de texture du revêtement cible basée sur cette corrélation.

**2.** Procédé informatique selon la revendication 1, dans lequel la prédiction d'au moins une caractéristique de texture du revêtement cible basée sur la corrélation comprend le fait de prédire, en utilisant au moins un calcul empirique, au moins une caractéristique de texture du revêtement cible basée sur la corrélation.

**3.** Système comprenant :

une base de données ; et un processeur en communication avec la base de données et programmé pour :

obtenir des données de réflectance spectrale d'un revêtement cible selon une pluralité d'angles de réflexion individuels, dans lequel les données de réflectance spectrale sont obtenues en utilisant un spectrophotomètre en des points choisis de l'espace correspondant aux angles de réflexion individuels ; calculer des données pseudo-électrostatiques à partir des données de réflectance spectrale, dans lequel le calcul des données pseudo-électrostatiques comprend le fait de traiter les données de réflectance spectrale obtenues aux angles de réflexion individuels comme des charges ponctuelles et d'appliquer la loi de Coulomb, et dans lequel le calcul se fait individuellement pour chaque longueur d'onde ; et générer d'après les données pseudo-électrostatiques une pigmentation de revêtement qui est la même ou sensiblement similaire en apparence au revêtement cible, dans lequel la génération de la pigmentation de revêtement comprend le fait de corréler de manière empirique les données pseudo-électrostatiques calculées pour le revêtement cible à une pluralité de données pseudo-électrostatiques connues calculées pour un ensemble de données empirique et de prédire au moins une caractéristique de texture du revêtement cible basée sur cette corrélation.

4. Système selon la revendication 3, comprenant en outre le spectrophotomètre en communication avec le processeur.

5. Support non transitoire lisible par ordinateur, comportant un logiciel destiné à faire exécuter par un processeur les opérations suivantes :

obtenir des données de réflectance spectrale d'un revêtement cible selon une pluralité d'angles de réflexion individuels, dans lequel les données de réflectance spectrale sont obtenues en utilisant un spectrophotomètre en des points choisis de l'espace correspondant aux angles de réflexion individuels ;

calculer des données pseudo-électrostatiques à partir des données de réflectance spectrale, dans lequel le calcul des données pseudo-électrostatiques comprend le fait de traiter les données de réflectance spectrale obtenues aux angles de réflexion individuels comme des charges ponctuelles et d'appliquer la loi de Coulomb, et dans lequel le calcul se fait individuellement pour chaque longueur d'onde ; et

générer d'après les données pseudo-électrostatiques une pigmentation de revêtement qui est la même ou sensiblement similaire en apparence au revêtement cible, dans lequel la génération de la pigmentation de revêtement comprend le fait de corréler de manière empirique les données pseudo-électrostatiques calculées pour le revêtement cible à une pluralité de données pseudo-électrostatiques connues calculées pour un ensemble de données empirique et de prédire au moins une caractéristique de texture du revêtement cible basée sur cette corrélation.

*FIG. 1*

*FIG. 2*

ALL ANGLES REPEATED FOR ADDITIONAL ILLUMINATORS
ADDITIONAL ANGLES POSSIBLE

Legend:
- AZIMUTHAL ANGLES
- IN-PLANE ANGLES

SPECULAR

75, 45, 25, 15, -15, 110

EP 3 066 436 B1

"FORCE" EXERTED BY 15° ANGLE

"FORCE" EXERTED BY AZIMUTHAL ANGLE

"FORCE" EXERTED BY AZIMUTHAL ANGLE

"FORCE" EXERTED BY AZIMUTHAL ANGLE

"FORCE" EXERTED BY AZIMUTHAL ANGLE

"FORCE" EXERTED BY BY 25° ANGLE

POINT OF MEASUREMENT

"FORCE" EXERTED BY 45° ANGLE

"FORCE" EXERTED BY 75° ANGLE

"FORCE" EXERTED BY 110° ANGLE

*FIG. 3*

EP 3 066 436 B1

"FORCE" EXERTED BY
BY 15° ANGLE

"FORCE" EXERTED
BY AZIMUTHAL
ANGLE

POINT OF MEASUREMENT

"FORCE" EXERTED BY
BY 110° ANGLE

*FIG. 4*

Oneway Analysis of Pairwise Force by
"Does this Contain a Gonioapparent Pigment?"

*FIG. 5*

FIG. 6

FIG. 7

**EP 3 066 436 B1**

**REFERENCES CITED IN THE DESCRIPTION**

**Patent documents cited in the description**

- US 61901493
- US 6539325 B1
- US 20080235224 A